# EUROPEAN PATENT APPLICATION

(11) **EP 1 870 415 A1**
(43) Date of publication of application: **26.12.2007**
(21) Application number: 07109094.8
(22) Date of filing: 29.05.2007
(51) Int. Cl.: C07D 495/04

(54) **Heterocyclic fused imidazolone, dioxolone, imidazolethione and dioxolethione monomers**

(30) Priority: 02.06.2006 US 446075
(71) Applicant: Air Products and Chemicals, Inc., Allentown, PA 18195-1501 (US)
(72) Inventor: Zahn, Steffen, Pennsburg, PA 18073 (US); Ford, Michael Edward, Trexlertown, PA 18087 (US)
(74) Representative: Schwabe - Sandmair - Marx

(57) **Abstract**

A method of making a compound of the formula shown below is disclosed: 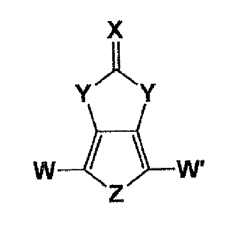 wherein Z is Se or S; Y is NH or O; X is O or S and W and W' are independently selected from the group consisting of hydrogen, -C=ONH₂, -C=ONHR', -C=ONR'R', -C≡N, and R' is C₁₋₆ alkyl and the resultant monomer compounds. The method includes contacting a 3,4-disubstituted thiophene or selenophene or disubstituted thiophene or selenophene derivatives with a carbonyl or thiocarbonyl containing compound selected from the group consisting of ureas, thioureas, carbonate esters, thionocarbonates, thiocarbonate esters, or orthocarbonates. The monomer compounds are suitable for forming polymers for use in a wide range of electronic applications.

## Description

### CROSS-REFERENCE TO RELATED APPLICATIONS

The subject matter of this application is related to U.S. Patent Application No. 11/446000, filed on even date herewith, and entitled "Electrically Conductive Polymers And Method Of Making Electrically Conductive Polymers"; the disclosure of which is hereby incorporated by reference.

### BACKGROUND OF THE INVENTION

The present invention is directed to monomers and methods for making monomers for forming electrically conductive polymers.

Electrically conducting polymers have developed into a material of choice for a variety of organic optoelectronics applications, Such applications for optoelectronics include polymeric light emitting diodes (thin film displays), solid state lighting, organic photovoltaics, advanced memory devices, organic field effect transistors, ultracapacitors, electroluminescent devices, printed electronics, conductors, lasers, and sensors.

One of the first of many electrically conducting polymers was polyacetylene and the discovery of conductivity in such polymer created substantial interest in other types of electrically conducting polymers. Recently, conjugated poly(thiophenes) and substituted thiophene derivatives have been discovered to have electrically conducting properties. A feature of these polymers is that they can be cast into films and doped with conventional p- and n-type dopants or the doped polymers can be cast into films and their electrical properties modified accordingly, thereby lending themselves suitable for use in a variety of optoelectronic applications.

Representative articles and patents illustrating known thiophene monomers and electrically conducting polymers including thiophene and derivatives thereof include the following:

US2004/00010115A1 to Sotzing discloses homopolymers and copolymers comprised of repeating units of thieno[3,4-*b*]thiophene for use in electroactive applications. The thieno[3,4-*b*]thiophene compounds disclosed in the US2004/00010115A1 includes the following structure:

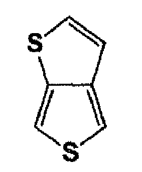

US2004/00010115A1 further discloses that copolymers can be formed with compounds including 3,4-ethylendioxythiophene, dithiophene, pyrrole, and benzothiophene.

US 6,645,401 to Giles et al. discloses conjugated polymers of dithienothiophene (DTT) with vinylene or acetylene connecting groups as suitable for producing semiconductors or charge transport materials useful in electrooptical and electronic devices including field effect transistors ("FET"), photovoltaic, and sensor devices.

US 6,585,914 to Marks discloses fluorocarbon-functionalized and/or heterocyclic modified poly(thiophenes) such as α, ω-diperfluorohexylsexithiophene for use in forming films which behave as n-type semiconductors. These poly(thiophenes) also can be used to form thin film transistors with FET mobility.

US 6,676,857 to Heeney et al. discloses polymers having polymerized units of 3-substituted-4-fluorothiophene as liquid crystal materials for use in semiconductors, charge transport materials, electrooptical field effect transistors, photovoltaic and sensor devices.

US 6,695,978 to Worrall et al. discloses polymers of benzo[b]thiophene and bisbenzo[b]-thiophene and their use as semiconductors and as charge transport materials in electrooptical devices.

US 6,709,808 to Lelental et al. discloses image forming materials incorporating electrically conductive polymers based upon pyrrole-containing thiophene polymers and aniline containing polymers.

US 2,487,051 to Mozingo et al. discloses preparation of 1 H-thieno[3,4-d]imidazol-2(3H)-one by contacting an aqueous solution of 3,4-diaminothiophene with phosgene. The material in US 2,487,051 is disclosed as an analog of biotin and no use of the material in any electronic application is disclosed.

European Patent EP 0 237 248 discloses a substituted imidazole anti-ulcer compound and a method of making the anti-ulcer compound. EP 0 237 248 discloses thieno[3,4-d]imidazol-2(3H)-thione as an intermediate compound during the manufacture of the anti-ulcer compound. No alternate use of the intermediate material is disclosed by EP 0 237 248.

The article, Hydrogenation of Compounds Containing Divalent Sulfur, J. Am. Chem. Soc. 1945, 67, 2092-2095 by Ralph Mozingo et al. (hereinafter "the Mozingo Article") discloses preparation of 1H-thieno[3,4-d]imidazol-2(3H)-one "in low yield" by contacting an aqueous solution of 3,4-diaminothiophene with phosgene.

While the above references include disclosures of known monomer compounds and methods of making these known monomer compounds, none of the above references discloses heterocyclic fused imidazolone, dioxolone, imidazolethione or dioxolethione monomers or methods of making the heterocyclic fused imidazolone, dioxolone, imidazolethione or dioxolethione monomers of the present invention. Further, known methods of forming monomers, particularly the method disclosed in US 2,487,051 and the Mozingo Article, utilize toxic chemicals, including phosgene, in the preparation of the various monomers, which results in dangerous reaction chemicals that are difficult to handle, store and dispose.

The disclosure of the foregoing patents, patent applications and publications is hereby incorporated by reference.

What is needed is a monomer capable of forming an electrically conductive polymer for a wide range of electronic applications that can be formed using readily available, easily handled reagents without the use of phosgene or other toxic reagents.

### BRIEF SUMMARY OF THE INVENTION

In one embodiment of the present invention, the invention provides a method of making a compound of the formula shown below is disclosed: 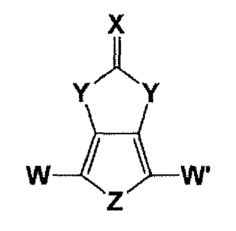 wherein Z is Se or S; Y is NH or O; X is O or S and W and W' are independently selected from the group consisting of hydrogen, -CO₂R', -C=ONR'R', and -C≡N; and R' is H or C₁₋₆ alkyl and the resultant monomer compounds. The method includes contacting a 3,4-disubstituted thiophene or selenophene or 3,4-disubstituted thiophene or selenophene derivatives with a carbonyl or thiocarbonyl containing compound selected from the group consisting of ureas, thioureas, carbonate esters, thionocarbonates, thiocarbonate esters, or orthocarbonates. The monomer compounds are suitable for forming polymers for use in a wide range of electronic applications.

Another embodiment of the present invention includes heterocyclic fused imidazolone, dioxolone, imidazolethione or dioxolethione monomer compounds according to the following formula: 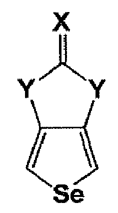 wherein Y is NH or O; and X is O or S,

Another embodiment of the present invention includes heterocyclic fused dioxolone, or dioxolethione monomer compounds according to the following formula: 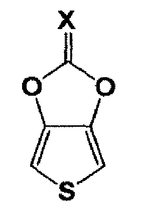 wherein X is S or O.

Another embodiment of the present invention includes heterocyclic fused compounds imidazolone, dioxolone, imidazolethione or dioxolethione including thieno[3,4-d]-1,3-dioxolan-2-one (1 a), selenolo[3,4-d]-1,3-dioxolan-2-one (1b) and 1 H-selenolo[3,4-d]imidazol-2(3H)-one (1c), and the thiocarbonyl compounds thieno[3,4-d]-1,3-dioxolan-2-thione (1d), selenolo[3,4-d]-1,3-dioxolan-2-thione (1e) and 1H-selenolo[3,4-d]imidazol-2(3N)-thione (1f), all shown by the following structures:

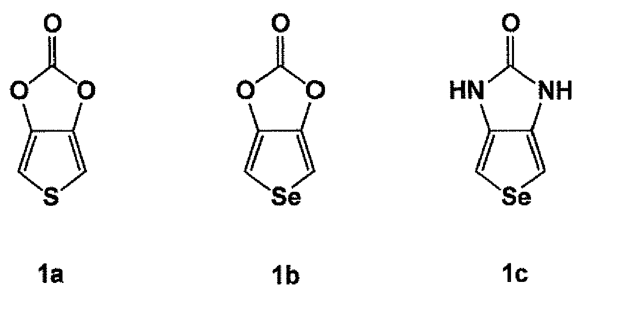

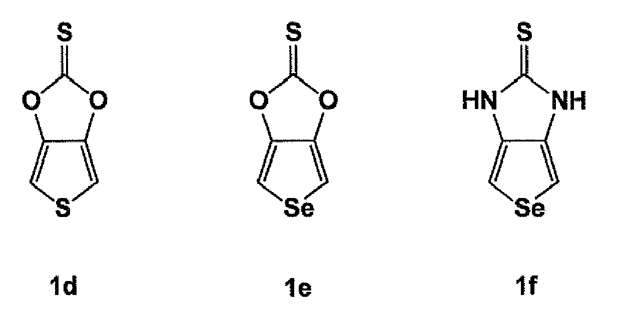

The invention includes heterocyclic fused imidazolone, dioxolone, imidazolethione and dioxolethione based monomers. Such monomers and polymers derived therefrom, find use in applications, including, but not limited to, hole injection materials, charge transport materials, semiconductors, and/or conductors, in optical, electrooptical or electronic devices, polymeric light emitting diodes (i.e., PLED), electroluminescent devices, organic field effect transistors (i.e., FET or OFET), flat panel display applications (e.g., LCD's), radio frequency identification (i.e., RFID) tags, printed electronics, ultracapacitors, organic photovoltaics (i.e., OPV), sensors, lasers, small molecule or polymer based memory devices, electrolytic capacitors, anti-corrosion coatings, or as hydrogen storage materials.

One advantage of a method according to an embodiment of the present invention includes preparation of previously unknown imidazolone, dioxolone, imidazolethione and dioxolethione monomers having an extended range of performance in many electronic applications.

Another advantage of a method according to an embodiment of the present invention includes preparation of heterocyclic fused imidazolone, dioxolone, imidazolethione and dioxolethione monomers without the necessity of using phosgene or other toxic reagents. That is, in one aspect of the invention, the monomers and processes for making the monomers can be substantially free of toxic reagents (e.g., the monomer and precursors thereby contain less than about _1_wt.% of phosgene, thiophosgene, among other toxic reagents). In addition, the heterocyclic fused imidazolone, dioxolone, imidazolethione and dioxolethione monomers may be prepared using readily available, easily handled reagents.

Still another advantage of a method according to an embodiment of the present invention includes preparation of heterocyclic fused imidazolone, dioxolone, imidazolethione and dioxolethione monomers in high yield, providing for an efficient, cost effective process. For example, the inventive process can produce a monomeric product having at least about 75wt.% monomer.

An advantage of an embodiment of the present invention is that heterocyclic fused imidazolone, dioxolone, imidazolethione and dioxolethione monomers and derivatives thereof may be used to produce conductive polymers having low work functions (e.g., polymers a conductivity of at least about 10⁻⁵ S/cm). For example, the present invention includes monomers for fabricating polymers suitable as a hole injecting material.

Another advantage of an embodiment of the present invention is that heterocyclic fused imidazolone, dioxolone, imidazolethione and dioxolethione monomers and derivatives thereof may be used to produce conducting polymers having a low band gap (e.g., a band gap of about <2.5 eV). For example, the present invention includes monomers for fabricating polymers suitable as transparent conductors.

Still another advantage of an embodiment of the present invention is that heterocyclic fused imidazolone, dioxolone, imidazolethione and dioxolethione monomers and derivatives thereof may be used to produce conducting polymers having a wide range of electronic applications.

Still another advantage of an embodiment of the present invention is that heterocyclic fused imidazolone, dioxolone, imidazolethione and dioxolethione monomers and derivatives thereof may be used to produce a hole injection material having desirable properties including a substantially identical work function level between the hole injection layer ("HIL") material and the light emitting layer in an electroluminescent device.

Still another advantage of an embodiment of the present invention is that heterocyclic fused imidazolone, dioxolone, imidazolethione and dioxolethione monomers and derivatives thereof may be used to produce an oxidized form of the polymer which results in desirable properties including the formation of a highly delocalized ionic polymer having high conductivity.

Still another advantage of an embodiment of the present invention is that heterocyclic fused imidazolone, dioxolone, imidazolethione and dioxolethione monomers and derivatives thereof may be used to produce solution processible materials.

Still another advantage of an embodiment of the present invention is that monomers based upon heterocyclic fused imidazolone, dioxolone, imidazolethione and dioxolethione and derivatives thereof may be used to produce an environmentally stable semiconducting polymer.

Other features and advantages of the present invention will be apparent from the following more detailed description of certain embodiments, taken in conjunction with the accompanying drawings which illustrate, by way of example, the principles of the invention.

### DETAILED DESCRIPTION OF THE INVENTION

This invention provides a method for making heterocyclic fused imidazolone and dioxolone monomers according to formula (1).

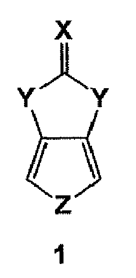

In formula (1), Z is S or Se; Y is NH or O; and X is O or S. The imidazolone, dioxolone, imidazolethione and/or dioxolethione according to formula (1) are fused heterocyclic compounds. "Fused" is defined as sharing a common bond within the ring between a thiophene or a selenophene and the imidazolone, dioxolone, imidazolethione and/or dioxolethione, thereby connecting the ring structures together.

Another embodiment of the present invention includes heterocyclic fused imidazolone, dioxolone, imidazolethione or dioxolethione monomer compounds according to the following formula:

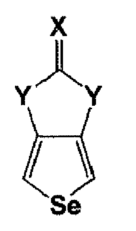

In the formula of the above embodiment, Y is NH or O; and X is O or S. The monomers of this embodiment include selenophene fused to the imidazolone, dioxolone, imidazolethione and/or dioxolethione groups.

Another embodiment of the present invention includes heterocyclic fused dioxolone, or dioxolethione monomer compounds according to the following formula:

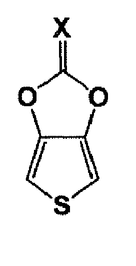

In the formula of the above embodiment, X is S or O. The monomers of this embodiment include thiophene fused to the dioxalone, and/or dioxolethione groups.

The present invention also provides for compositions of monomer compounds according to structures (1a - 1f).

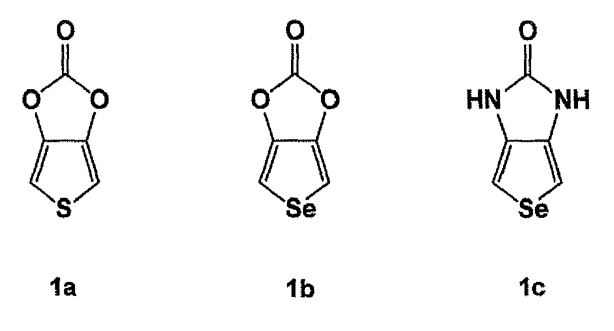

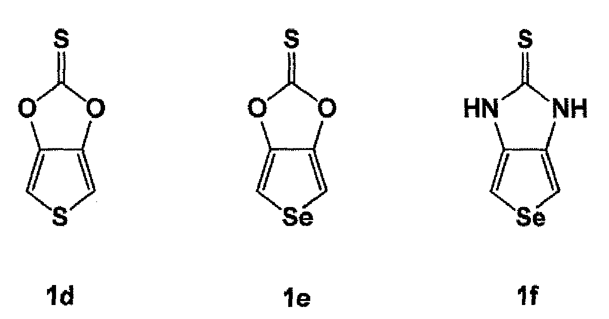

The conductive polymers formed from a process of one embodiment of the invention possess functionalities that enable solid state engineering through hydrogen bonding interactions. Depending of the choice of Y and X of formula M1 the structural motifs generated through hydrogen bonding interaction may vary greatly, The structural motifs may be varied by adding additives or other hydrogen bonding enabling molecules as described in the open literature. For examples see, Journal of the American Chemical Society, 1996, 118, 4018-4029 and references therein (hereby incorporated by reference). Monomers for formation of the polymer according to the present invention, such as 1H-thieno[3,4-d]imidazol-2(3H)-one, form a structural motif that may be characterized as a linear tape. The linear motif is maintained through out the polymerization leading to films of polymers that are highly ordered as evident by their very high gloss. Monomers which form conductive polymers upon polymerization but lack solid state engineering through hydrogen bonding appear flat and display high surface roughness values.

Monomer compounds according to the present invention may be obtained by reaction of a first reactant compound, such as a 3,4-dihydroxy- or 3,4-diamino-substituted thiophene or selenophene, with a second reactant compound, such as a carbonyl or thiocarbonyl containing compound, and reacting the first reactant compound with the second reactant compound to form the desired monomer. A 3,4-dihydroxy- or 3,4-diamino substituted heterocycle suitable for use as the first reactant compound may include 3,4-dihydroxythiophene. The preparation of 3,4-dihydroxy- or 3,4-diamino substituted heterocycles may take place according any suitable preparation method, such as the method disclosed in U.S. Patent No. 6,869,729 to Pope et al., example 1 or by the method disclosed by Q. T. Zhang and J. M. Tour, J. Am. Chem. Soc. 1997, 119, 9624-9631; both hereby incorporated by reference. The 3,4-diaminothiophene may be utilized as a first reactant compound precursor obtained as a dihydrochloride salt. The dihydrochloride salt may be readily stored and handled. However, the free base of the 3,4 diaminothiophene as the first reactant compound is useful for practicing the method of this invention. The use of the free base is advantageous because it does not have to be generated or stored prior to practicing the invention. In one embodiment of the invention, the 3,4 diaminothiophene is generated in situ by inclusion of an appropriate organic or inorganic base with the first reactant compound precursor (e.g. 3,4-diaminothiophene dihydrochloride) in the reaction medium. Examples of suitable bases can comprise at least one member selected from the group consisting of organic bases such as triethylamine, pyridine, 2-(dimethylamino)pyridine, 4-(dimethylamino)pyridine, 1,8-bis(N,N-dimethylamino)naphthatene, polymer-bound 4-(dimethylamino)pyridine, and N,N-diisopropylethylamine, and inorganic bases such as sodium carbonate, sodium bicarbonate, potassium carbonate, potassium bicarbonate, potassium phosphate, sodium hydroxide, potassium hydroxide, cesium carbonate, and cesium hydroxide, among others.

The second reactant compound is a compound capable of fusing to the thiophene or selenophene to form a imidazolone, dioxolone, imidazolethione and/or dioxolethione. Suitable second reactant compounds with which the first reactant compound is reacted may be a urea, represented by formula (2), a carbonate ester, represented by formula (3), or an orthocarbonate, represented by formula (4).

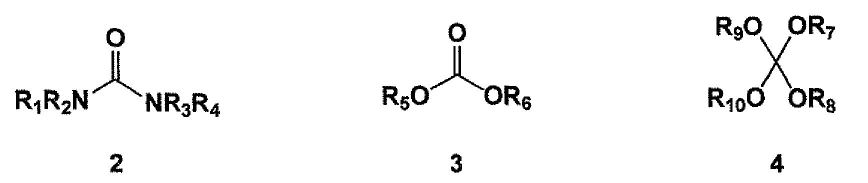

R₁, R₂, R₃ and R₄ are independently chosen from the group consisting of hydrogen; C₁ - C₁₂ linear, cyclic, and branched alkyl, alkenyl, aryl, aralkyl, and alkaryl groups; imidazole, triazole, or benzotriazole rings or portions thereof; C₂ - C₆ linear or branched alkylenes; and combinations thereof. Suitable urea compounds for use as second reactant compounds include, but are not limited to, urea, N,N'-dimethylurea, N,N-dimethylurea, ethyleneurea, 1,1' -carbonyldiimidazole, 1,1'-carbonylbis(2-methylimidazole), 1,1'-carbonyldi(1,2,4-triazole), 1,1'-carbonylbisbenzotriazole, and 1,1'-carbonyldipiperidine.

R₅ and R₆ are independently chosen from the group consisting of C₁ - C₁₂ linear, cyclic, and branched alkyl, alkenyl, aryl, aralkyl, and alkaryl groups; C₂ - C₆ alkylene and branched alkylene groups and combinations thereof. Suitable carbonate esters for use as the second reactant compound include, but are not limited to, diethyl carbonate, ethylene carbonate, and propylene carbonate.

R₇ - R₁₀ are independently chosen from the group consisting of C₁ - C₈ linear, cyclic, and branched alkyl, aryl, aralkyl, and alkaryl groups and combinations thereof. Suitable orthocarbonates for use as the second reactant compound include, but are not limited to, tetramethyl orthocarbonate, tetraethyl orthocarbonate, and tetrapropyl orthocarbonate.

In an alternate embodiment of the present invention, the second reactant compound may include a thiocarbonyl compound with which the first reactant compound, typically a 3,4-disubstituted heterocycle, is reacted. Suitable thiocarbonyl compounds for use as the second reactant compound may include thiourea, represented by formula (5), or a thionocarbonate, represented by formula (6).

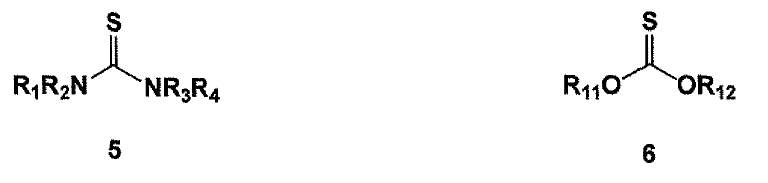

R₁, R₂, R₃ and R_{4,} are as before, i.e., independently chosen from the group consisting of hydrogen; C₁ - C₁₂ linear, cyclic, and branched alkyl, alkenyl, aryl, aralkyl, and alkaryl groups; imidazole, triazole, or benzotriazole rings or portions thereof; C₂ - C₆ linear or branched alkylenes; and combinations thereof. Suitable thioureas for use as the second reactant compound include, but are not limited to, thiourea, N,N'-dimethylthiourea, N,N-dimethylthiourea, 1,1'-thiocarbonyldiimidazole, 1,1'-thiocarbonylbis(2-methylimidazole), 1,1'-thiocarbanyldi(1,2,4-triazole), 1,1'-thiocarbonylbisbenzotriazole, and 1,1'-thincarbanyldipiperidine.

R₁₁ and R₁₂ are independently chosen from the group consisting of C₁ - C₁₂ linear, cyclic, and branched alkyl, alkenyl, aryl, aralkyl, and alkaryl groups; C₂ - C₆ alkylene and branched alkylene groups; 2-pyridyl; and 4-pyridyl. Examples of suitable thionocarbonate esters include, but are not limited to, dimethyl thionocarbonate, diethyl thionocarbonate, ethylene thionocarbonate, di-2-pyeidyl thionocarbonate, di-4-pyridyl thionocarbonate and combinations thereof.

In one embodiment of the method of the present invention, a dihydrochloride salt, such as 3,4-diaminothiophene dihydrochloride, is added to the reaction mixture to prepare the first reactant compound. A base can be included to prepare the first reactant compound from the salt. Examples of suitable bases for this purpose include, but may not be limited to, organic bases such as triethylamine, pyridine, 2-(dimethylamino)pyridine, 4-(dimethylamino)pyridine, polymer-bound 4-(dimethylamino)pyridine, and N,N-diisopropylethylamine, and inorganic bases such as sodium carbonate, sodium bicarbonate, potassium carbonate, potassium bicarbonate, sodium hydroxide, and potassium hydroxide. Bases that are insoluble in the reaction mixture, such as sodium carbonate or sodium bicarbonate, are useful.

A variety of organic solvents may be used as the reaction mixture for preparation of the fused imidazolone, dioxolone, imidazolethione or dioxolethione monomer compounds. Examples of suitable organic solvents include, but may not be limited to, 1,3-dimethyl-2-imidazolidinone, 1,3-dimethyl-3,4,5,6-tetrahydro-2(1H)-pyrimidinone, ethylene glycol, propylene glycol, 1,2-dimethoxyethane, tetrahydrofuran, and mixtures thereof. A desirable organic solvent comprises 1,3-dimethyl-2-imidazolidinone. The reaction temperature is sufficiently high to react the first reactant compound with the second reactant compound to form the desired fused imidazolone, dioxolone, imidazolethione or dioxolethione monomer. The reaction temperatures may be in the range of from about 80°C to about 175°C, typically from about 85°C to about 150°C, and more typically from about 90°C to about 125°C. The optimum conditions will depend on the reactor configuration, the solvents employed, and other variables.

Monomers that may be prepared according to the method of the present invention include, but are not limited to, 1 H-thieno[3,4-d]imidazol-2(3H)-one, 1 H-selenolo[3,4-d]imidazol-2(3H)-one, thieno[3,4-d]-1,3-dioxolan-2-one, thieno[3,4-d]-1,3-dioxolan-2-thione, selenolo[3,4-d]-1,3-dioxolan-2-one, 1H-thieno[3,4-d]imidazal-2(3H)-thione, 1 H-selenolo[3,4-d]imidazol-2(3H)-thione, and selenolo[3,4-d]-1,3-dioxolan-2-thione. The structures for these monomers are as follows:

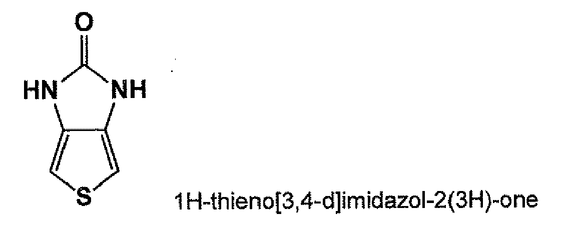

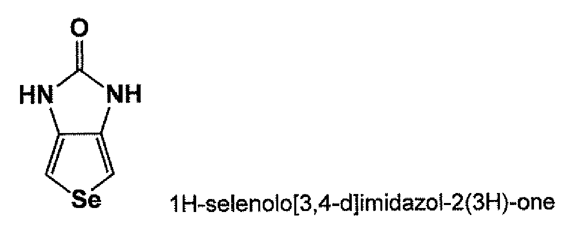

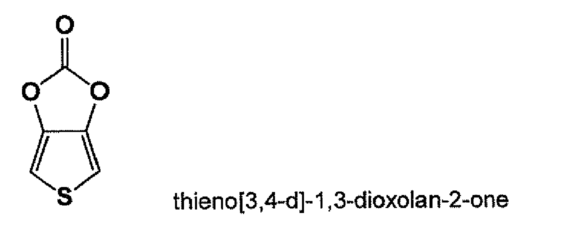

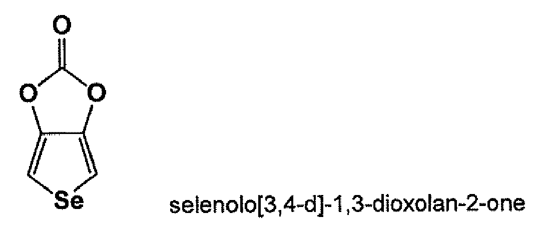

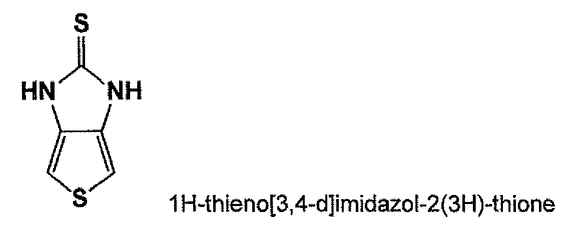

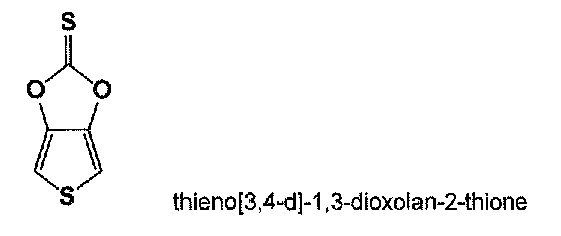

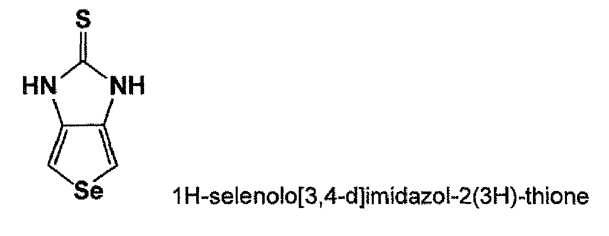

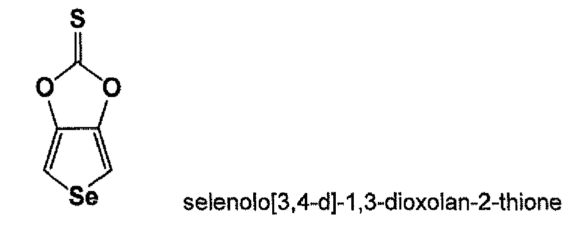

A method of making heterocyclic fused imidazolone, dioxolone, imidazolethione, and dioxolethione according to an embodiment of the present invention may include one or more steps in a single reaction mixture including the following mechanism:

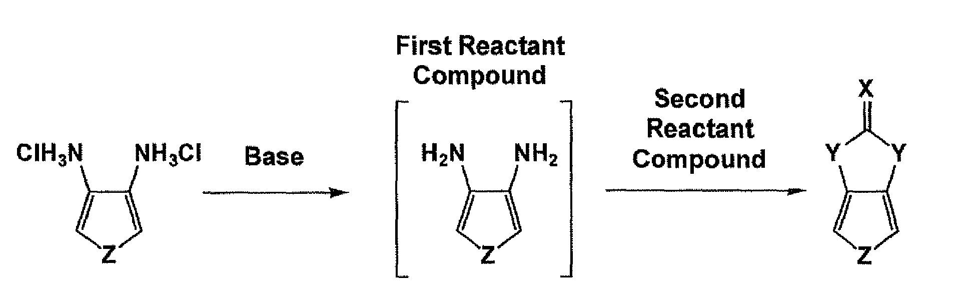

In the compounds in the above reaction, Z is S or Se; Y is NH or O; and X is O or S. The method includes a preparation of the first reactant compound with a base, such as sodium carbonate, triethylamine or 4-(dimethylamino) pyridine, which is used to liberate the first reactant compound, shown as 3,4-diaminothiophene (or 3,4-diaminoselenophene). While the first reactant compound shown above is 3,4-diaminothiophene (or 3,4-diaminoselenophene) and the first reactant compound precursor is 3,4-diaminothiophene (or 3,4-diaminoselenophene) dihydrochloride, the first reactant compound may be any suitable compound capable of reacting with the second reactant compound to form the heterocyclic fused imidazolone, dioxolone, imidazolethione or dioxolethione based monomers of the present invention. For example, in the formation of dioxolone or dioxolethione, the corresponding 3,4-dihydroxythiophene may be utilized in place of the 3,4-diaminothiophene. The first reactant compound is reacted with the second reactant compound and the heterocyclic fused imidazolone, dioxolone, imidazolethione or dioxolethione based monomer according to the present invention is formed.

The method of the present invention may include synthesis of 1 H-thieno[3,4-d]imidazol-2(3H)-one in a single reaction mixture. The process according to the present invention for the synthesis of 1 H-thieno[3,4-d]imidazol-2(3H)-one includes the mechanism shown as follows:

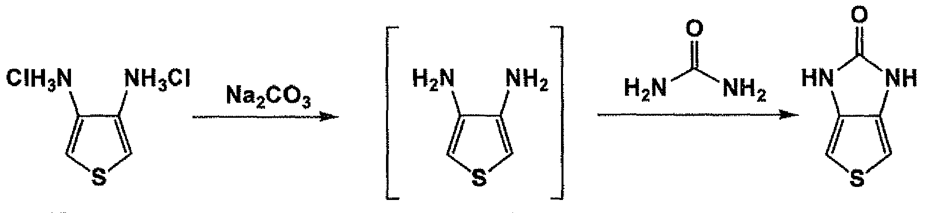

This first reactant compound, the second reactant compound, solvent and base are present in the reaction mixture. In the reaction above, 3,4-diaminothiophene dihydrochloride reacts in the presence of a base, shown as sodium carbonate (Na₂CO₃), to form 3,4-diaminothiophene, which reacts with the second reactant compound, shown above as urea, to form the 1 H-thieno[3,4-d]imidazol-2(3H)-one.

The method of the present invention also includes synthesis of imidazolone-based selenophene derivatives. In this embodiment of the present invention, 3,4-diaminoselenophene dihydrochloride is reacted as follows:

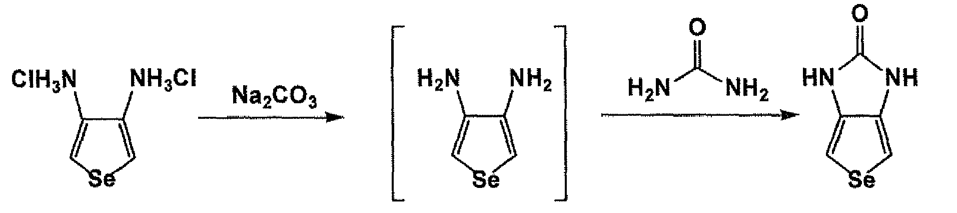

In the reaction above, 3,4-diaminoselenophene dihydrochloride reacts in the presence of a base, shown above as sodium carbonate (Na₂CO₃), to form 3,4-diaminoselenophene, which reacts with the second reactant compound, shown above as urea, to form the 1H-selenolo[3,4-d]imidazol-2(3H)-one.

To convert 3,4-dihydroxythiophene or 3,4-dihydroxyselenophene reactant compounds to the corresponding dioxolone a similar mechanism is utilized. In one embodiment of the present invention, 3,4-dihydroxythiophene is reacted in a single step, as follows:

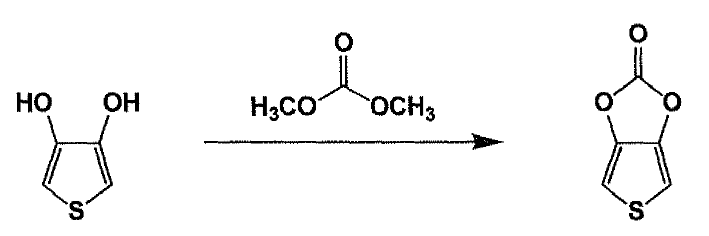

In this reaction 3,4-dihydroxythiophene reacts with the second reactant compound, shown above as dimethyl carbonate, to form the thieno[3,4-d]-1,3-dioxolan-2-one.

In another embodiment of the present invention, 3,4-dihydroxythiophene is reacted as follows:

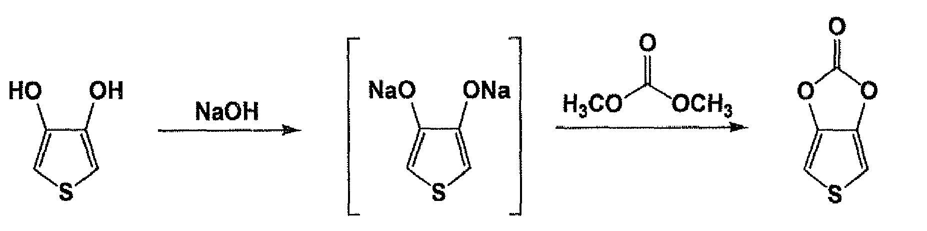

In this reaction, 3,4-dihydroxythiophene reacts in the presence of a base, shown above as sodium hydroxide (NaOH) to form an intermediate sodium salt, shown above as the disodium salt, which reacts with the second reactant compound, shown above as dimethyl carbonate, to form the thieno[3,4-d]-1,3-dioxolan-2-one. In order to produce the selenolo[3,4-d]-1,3-dioxolan-2-one, similar reaction mechanisms to those for the conversion of 3,4-dihydroxythiophene to thieno[3,4-d]-1,3-dioxolan-2-one are utilized, except that a 3,4-dihydroxyselenophene is utilized in the place of the 3,4-dihydroxythiophene shown previously.

In order to produce the imidazolethione or dioxolethione monomers of the present invention, the following reaction may be utilized:

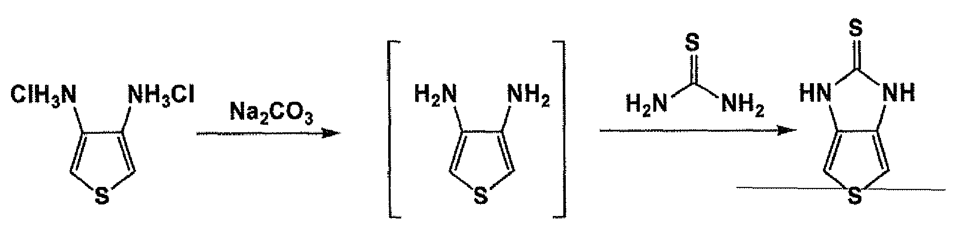

In this reaction 3,4-diaminothiophene dihydrochloride reacts in the presence of a base, shown above as sodium carbonate (Na₂CO₃), to form 3,4-diaminothiophene, which reacts with the second reactant compound, shown above as thiourea, to form the 1H-thieno[3,4-d]-imidazol-2(3H)-thione. In order to produce the 1 H-selenolo[3,4-d]imidazol-2(3H)-thione, a similar reaction mechanism to the conversion of 3,4-diaminothiophene dihydrochloride to 1H-thieno[3,4-d]-imidazol-2(3H)-thione is utilized, except a 3,4-diaminoselephene dihydrochloride is utilizes in the place of the 3,4-diaminothiophene dihydrochloride shown previously. Likewise, in order to produce thieno[3,4-d]-1,3-dioxolan-2-thione, a similar mechanism to the conversion of 3,4-dihydroxythiophene to thieno[3,4-d]-1,3-dioxolan-2-one is utilized, except that the second reactant compound is selected to include a thiocarbonate ester.

In an alternate embodiment of the method of the present invention, derivatives of 3,4-diaminothiophene, 3,4-dihydroxythiophene, 3,4-diaminoselenophene, and 3,4-dihydroxyselenophene may be used as the first reactant compound. Suitable derivative compounds for use with this embodiment of the invention include, but are not limited to, 3,4-diaminothiophene, 3,4-dihydroxythiophene, 3,4-diaminoselenophene, and/or 3,4-dihydroxyselenophene having carboxylate groups, nitrile groups and/or carboxamide groups attached to the 2- and 5- positions in the thiophene or selenophene ring. The derivative first reactant compounds is reacted with a second reactant compound capable of fusing to the 3,4-diaminothiophene, 3,4-dihydroxythiophene, 3,4-diaminoselenophene, and/or 3,4-dihydroxyselenophene derivative to form a imidazolone, dioxolone, imidazolethione, and dioxolethione represented by the following formula:

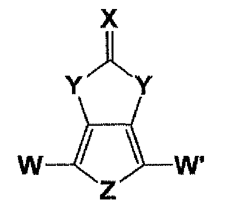

In the above compound, Z is S or Se; Y is NH or O; X is O or S; and W and W' may include any suitable derivative group or atom. Suitable derivative groups include, but are not limited to carboxylate, nitrile and/or carboxamide. For example, in the above formula W and W' may be independently selected from the group consisting of hydrogen, -CO₂R', -C=ONR'R' , and -C=N; and R' is H or C₁₋₆ alkyl. Subsequent to formation of the imidazolone, dioxolone, imidazolethione, and dioxolethione, the derivative groups (i.e., W and W') may be removed to provide the corresponding 2,5-unsubstituted imidazolone, dioxolone, imidazolethione, and dioxolethione.

In one embodiment of the method of the present invention, a 2,5-dicarboxylated 3,4-dihydroxythiophene (obtained, e.g., by the method of W. A. Feld, et. al, Synthetic Communications, 1996, 26, 2205-2212 or the method of B. D. Tilak, et. al., Tetrahedron, 1967, 23, 2437-2241) or a 2,5-dicarboxylated 3,4-dihydroxyselenophene or ester thereof (obtained, e.g., by the method of M. P. Cava, et. al., Organic Letters, 2001, 26, 4283-4285; hereby incorporated by reference) is reacted with a second reactant compound capable of fusing to the 3,4-dihydroxythiophene or 3,4-dihydroxyselenophene to form a dioxolone or a dioxolethione represented by the following formula: 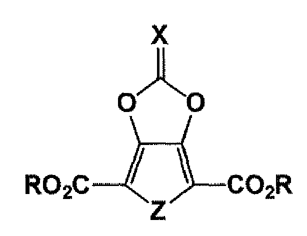 wherein Z is S or Se; and X is O or S; and R is hydrogen, or C₁ - C₆ linear, branched or cyclic alkyl group. Subsequent to formation of the dioxolone or dioxolethione, the carboxylate groups are removed by any of the procedures in the foregoing three references to provide the corresponding 2,5-unsubstituted dioxolones or dioxolethiones.

The method of the present invention may include making a fused dioxolone or dioxolethione according to an embodiment of the present invention in a multi-step procedure as follows:

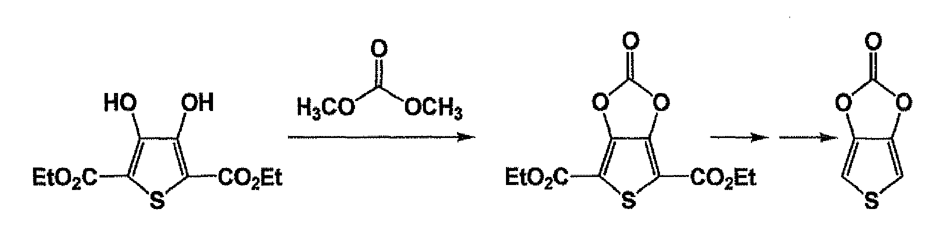

In the reaction shown above, diethyl 3,4-dihydroxythiophene-2,5-dicarboxylate reacts with the second reactant compound, shown as dimethyl carbonate, to form the diethyl thieno[3,4-d]-1,3-dioxolan-2-one 2,5-dicarboxylate.

Subsequent to formation of the dioxolone or dioxolethione ring, the carboxylate groups are removed by any suitable procedure, such as the procedures contained in the foregoing references to Feld, et. al., Tilak, et. al., or Cava, et. al. (hereby incorporated by reference), to provide the corresponding thieno[3,4-d]-1,3-dioxolan-2-one.

In another embodiment of the present invention, derivatives of the heterocyclic fused imidazolone, dioxolone, imidazolethione, and dioxolethione are formed prior to or after the formation of the fully aromatic fused heterocyclic monomer. Monomer derivatives according to the present invention may include compounds having the following formula:

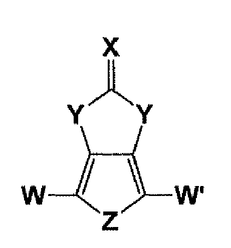

In the above derivative compound above, Z is S or Se; Y is NH or O; X is O or S; and W and W' may include any suitable group or atom. In one embodiment W and W' are independently selected from the group consisting of hydrogen, halogen atoms, MgCl, MgBr, Mgl, ZnCl, ZnBr, Znl, Sn(R')₃, boronic acid, boronic ester, -CH=CHR", -OC₁₋₆ alkyl, -COOC₁₋₆alkyl, -S-COR"', -COR"', -C≡CH, and polymerizable aromatic groups. R' is C₁₋₆ alkyl or -OC₁₋₆ alkyl. R" is H or C₁₋₆ alkyl. R"' is H or C₁₋₆ alkyl. Suitable polymerizable aromatic groups may include phenyl, naphthalene, pyrrole, dithiophene, thienothiophene, thiophene, and other polymerizable aromatic group containing moieties.

The polymerization and the resulting polymer can be controlled by selecting moieties W and W' having the desired polymerization reaction and desired resultant polymer. Carbon-carbon bond forming reactions may be completed following any suitable method. Methods suitable for use with the monomer of the present invention include, but are not limited to the Suzuki Reaction, the Yamamoto Reaction, the Heck Reaction, the Stille Reaction, the Sonogashira-Hagihara Reaction, the Kumada-Corriu Reaction, the Riecke Reaction, and the McCullogh Reaction.

Derivatives according to the present invention may include homopolymers and copolymers in which W and W' are H. In another embodiment, the compounds of the present invention may include homopolymer and copolymers wherein W, and W' are Br. In still another embodiment, the compounds of the present invention may include homopolymer and copolymers wherein W, and W' are trialkylstannyl.

Many of the derivatives of the respective monomers where W and W' are other than H are formed post-formation of the monomers. In one such post-reaction, one or both hydrogen atoms may be replaced with other functional groups such as bromide or trialkylstannyl groups. The replacement of the hydrogen atoms may take place using any reaction mechanism suitable for use with heterocyclic ring structures. In an alternate embodiment, the W and/or W' containing derivatives may be formed prior to converting thiophene to the first reaction product (e.g., 3,4-dihydroxythiophene) and then undergoing a reaction procedure shown above for the conversion of 3,4-dihydroxythiophene or 3,4-dihydroxyselenophene derivatives to the imidazolone, dioxolone, imidazolethione or dioxolethione based monomers where the W and W' are compatible with the chemistry outlined above.

### EXAMPLES

The following examples are provided to illustrate various embodiments and comparisons and are not intended to restrict the scope of the invention.

### Example 1

The compound 1 H-thieno[3,4-d]imidazol-2(3H)-one was prepared in a single reaction mixture in accordance with the method of the present invention. In Example 1, triethylamine was utilized as a base to liberate 3,4-diaminothiophene from the dihydrochloride salt. Urea was used as the carbonyl containing second reactant compound.

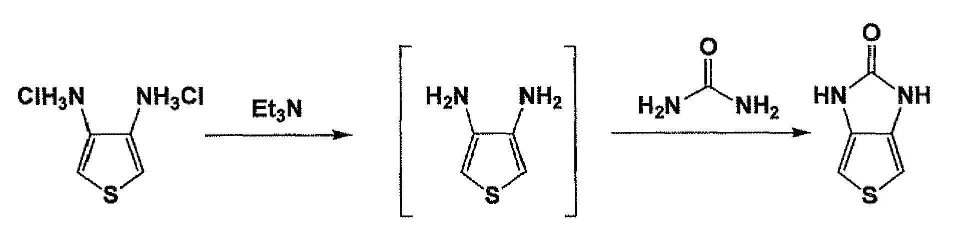

An amount of 0.1922 gm (1.028 x 10⁻³ mole) 3,4-diaminothiophene dihydrochloride ("DAT-HCl") and 0.1866 gm (3.11 x 10⁻³ mole; 3.02 equiv., based on DAT-HCl) urea were added to a 50 mL three-necked flask equipped with magnetic stirring and a reflux condenser. The flask was purged with nitrogen for 15 minutes, after which 20 mL of 1,3-dimethyl-2-imidazolidinone ("DMI") was added via syringe with stirring. The contents were warmed to 50°C, at which point 0.45 mL (0.327 gm, 3.23 × 10⁻³ mole; 3.14 equiv., based on DAT-HCl) triethylamine was added via syringe. The temperature of the flask was raised to 115 - 120°C and maintained in that temperature range for 4 hours. After cooling and filtration, gas chromatography ("GC") analysis showed complete conversion of 3,4-diaminothiophene; selectivity to 1 H-thieno[3,4-d]imidazol-2(3H)-one was >98%. Components were identified by gas chromatography ("GC") and mass spectrometry ("MS"), and by comparison of retention times with those of known standards.

### Comparative Example 1B

Comparative Example 1 B includes an attempt to prepare 1H-thieno[3,4-d]imidazol-2(3H)-one in a single reaction mixture having the components utilized in Example 1. Comparative example 1B utilizes a reaction mixture that is not heated above room temperature.

The procedure of Example 1 was repeated, with use of ethanol as the solvent. in addition, the reaction mixture was not heated and was stirred at room temperature for 58 hrs after addition of the triethylamine. GC analysis at the end of that time showed that none of the 3,4-diaminothiophene had been converted into 1 H-thieno[3,4-d]imidazol-2(3H)-one.

### Example 2

1 H-thieno[3,4-d]imidazol-2(3H)-one was prepared in a single reaction mixture in accordance with the method of the present invention. Example 2 uses an alternate base from Example 1, 4-(dimethylamino)-pyridine, as the base to liberate 3,4-diaminothiophene from the dihydrochloride salt. In addition, Example 2 utilizes 1,1'-carbonyldiimidazole as the second reactant compound.

An amount of 0.1934 gm (1.034 x 10⁻³ mole) 3,4-diaminothiophene dihydrochloride (DAT-HCl), 0.4862 gm (3.001 × 10⁻³ mole; 2.90 equiv., based on DAT-HCl) 1,1'-carbonyldiimidazole, and 0.3957 gm (3.243 x 10⁻³ mole; 3.14 equiv., based on DAT-HCl) 4-(dimethylarnino)pyridine were added to a 50 mL three-necked flask equipped with magnetic stirring and a reflux condenser. The flask was purged with nitrogen for 15 minutes, after which 20 mL of 1,3-dimethyl-2-imidazolidinone (DMI) was added via syringe with stirring. The temperature of the flask was raised to 115 -120°C and maintained in that temperature range for 4 hours. After cooling and filtration, GC analysis showed complete conversion of 3,4-diaminothiophene; selectivity to 1 H-thieno[3,4-d]imidazol-2(3H)-one was >98%.

### Example 3

The compound 1 H-thieno[3,4-d]imidazol-2(3H)-one was prepared in a single reaction mixture in accordance with the method of the present invention. Example 3 includes the use of sodium carbonate as the base to liberate 3,4-diaminothiophene from the dihydrochloride salt, and use of 1,1'-carbonyldiimidazole as the second reactant compound.

The procedure of Example 2 was followed with 0.1981 gm (1.059 × 10⁻³ mole) 3,4-diaminothiophene dihydrochloride ("DAT-HCl"), and use of 0.4868 gm (3.005 × 10⁻³ mole; 2.84 equiv., based on DAT-HCl) 1,1'-carbonyldiimidazole as the carbonyl containing reactant and 0.6591 gm (6.218 x 10⁻³ mole; 5.87 equiv., based on DAT-HCl) anhydrous sodium carbonate as the included base. After cooling and filtration, GC analysis showed complete conversion of 3,4-diaminothiophene; selectivity to 1H-thieno[3,4-d]imidazol-2(3H)-one was >98%.

### Example 4

The compound 1 H-thieno[3,4-d]imidazol-2(3H)-one was prepared in a single reaction mixture in accordance with the method of the present invention. Example 4 includes the use of sodium carbonate as the base to liberate 3,4-diaminothiophene from the dihydrochloride salt, and use of 1,1'-carbonyldiimidazole as the second reactant compound wherein the amount of reactants utilized was increased in comparison to Examples 1-3.

An amount of 1.8890 gm (10.102 × 10⁻³ mole) 3,4-diaminothiophene dihydrochloride (DAT-HC!), 3.2668 gm (20.165 x 10⁻³ mole; 2.00 equiv., based on DAT-HCl) 1,1'-carbanyldiimidazole, and 8.5445 gm (80.608 x 10⁻³ mole; 7.98 equiv., based on DAT-HCl) anhydrous sodium carbonate were added to a 100 mL three-necked flask equipped with magnetic stirring and a reflux condenser. The flask was purged with nitrogen for 15 minutes, after which 60 mL of 1,3-dimethyl-2-imidazolidinone (DMI) was added via syringe with stirring. The temperature of the flask was raised to 125°C and maintained at that temperature for 3 hours. After cooling and filtration, GC analysis showed complete conversion of 3,4-diaminothiophene; selectivity to 1H-thieno[3,4-d]imidazol-2(3H)-one was >98%.

### Example 5

The compound 1H-thieno[3,4-d]imidazol-2(3H)-one was prepared in a single reaction mixture in accordance with the method of the present invention. This example illustrates use of ethylene carbonate as the carbonyl containing compound.

The procedure of example 3 was followed, with use of 0.1972 gm (1.054 x 10-³ mole) DAT-HCl and 0.2870 gm (3.261 x 10-3 mole; 3.09 equiv., based on DAT-HCl) ethylene carbonate in place of the 1,1'-carbonyldiimidazole. After cooling and filtration, GC analysis showed 79% conversion of 3,4-diaminothiophene; selectivity to 1H-thieno[3,4-d]imidazol-2(3H)-one was >90%.

### Example 6

The compound thieno[3,4-d]-1 ,3-dioxolan-2-one is prepared in a single step procedure in a manner in accordance with the present invention.

The procedure of Example 3 is followed with 0.122 gm (1.0 × 10⁻³ mole) 3,4-dihydroxythiophene (DHT) in place of 3,4-diaminothiophene dihydrochloride, and use of 0.486 gm (3.0 × 10⁻³ mole; 3.0 equiv., based on DHT) 1,1'-carbonyldiimidazole as the carbonyl containing reactant. After cooling and filtration, analysis will show the presence of thieno[3,4-d]-1,3-dioxolan-2-one.

### Example 7

A solution containing 1 H,3H-Thieno[3,4-d]imidazol-2-one and the lron(lll) salt of Toluene-4-sulfonic acid in anhydrous n-butanol was prepared. The solution was 0.1 M in 1 H,3H-Thieno[3,4-d]imidazol-2-one and 0.16 M in the Iron(III) salt of Toluene-4-sulfonic acid. The solution was stored in a closed vial to prevent loss of solvent over time. However, the vial was opened for 10 minutes daily to allow fresh air to enter. Over the course of a week the solution did not change color nor yield any visible precipitate suggesting that 1 H,3H-Thieno[3,4-d]imidazol-2-one is stable in the presence of its iron(lll) oxidant.

While the invention has been described with reference to certain embodiment, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims.

## Claims

1. A method for making a monomer for forming an electrically conductive polymer comprising:
providing a first reactant comprising at least one compound selected from the group consisting of 3,4-diaminothiophene, 3,4-dihydroxythiophene, 3,4-diaminoselenophene, 3,4-dihydroxyselenophene, derivatives of 3,4-diaminothiophene, derivatives of 3,4-dihydroxythiophene, derivatives of 3,4-diaminoselenophene, derivatives of 3,4-dihydroxyselenophene, and combinations thereof;
providing a second reactant comprising at least one compound selected from the group consisting of a urea containing compound, a carbonate ester containing compound, an orthoester containing compound, a thiourea containing compound, a thionocarbonate containing compound and combinations thereof;
contacting the first reactant compound and second reactant compound to form a reaction mixture;
heating the reaction mixture to a temperature sufficient to produce a monomer comprising the following formula: 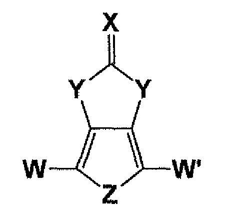
wherein Z comprises Se or S; Y comprises NH or O; X comprises O or S and W and W' comprise at least one member independently selected from the group consisting of hydrogen, -CO₂R' -C=ONR'R' , and -C≡N; and R' is H or C₁₋₆ alkyl.

2. The method of claim 1, wherein the second reactant compound comprises at least one urea compound selected from the group consisting of carbonyldiimidazole, carbonyldi(1,2,4-triazole) and combinations thereof.

3. The method of claim 1, wherein the second reactant compound comprises at least one carbonate ester compound selected from the group consisting of dimethyl carbonate, diethyl carbonate, ethylene carbonate, propylene carbonate and combinations thereof.

4. The method of claim 1, wherein the second reactant compound comprises at least one orthocarbonate compound selected from the group consisting of tetramethyl orthocarbonate, tetraethyl orthocarbonate, tetrapropyl orthocarbonate and combinations thereof.

5. The method of claim 1, wherein the second reactant compound comprises at least one thiourea containing compound is selected from the group consisting of thiourea, N,N'-dimethylthiourea, 1,1'-thiocarbonyldiimidazole, and combinations thereof.

6. The method of claim 1, wherein the second reactant compound comprises at least one thionocarbonate containing compound selected from the group consisting of di-2-pyridiyl thionocarbonate, di-4-pyridyl thionocarbonate and combinations thereof.

7. The method of claim 1, wherein the heating step includes heating to a temperature from about 80°C and about 175°C.

8. The method of claim 7, wherein the heating step includes heating to a temperature from about 90°C and about 125°C.

9. The method of claim 1, wherein the contacting is performed in the presence of at least one solvent.

10. The method of claim 9, wherein the solvent comprises at least one member selected from the group consisting of 1,3-dimethyl-2-imidazolidinone, 1,3-dimethyl-3,4,5,6-tetrahydro-2(1H)-pyrimidinone, ethylene glycol, propylene glycol, 1,2-dimethoxyethane, tetrahydrofuran, and combinations thereof.

11. The method of claim 1, further comprising providing a precursor of the first reactant compound and a base material to convert the first reactant compound precursor into the first reactant compound.

12. The method claim 11, wherein the first reactant compound precursor comprises at least one member selected from the group consisting of 3,4-diaminothiophene dihydrochloride or 3,4-diaminoselenophene dihydrochloride.

13. The method of claim 12, wherein the base material comprises at least one member selected from the group consisting of triethylamine, pyridine, 2-(dimethylamino)pyridine, 4-(dimethylamino)pyridine, polymer-bound 4-(dimethylamino)pyridine, and N,N-diisopropylethylamine, and inorganic bases such as sodium carbonate, sodium bicarbonate, potassium carbonate, potassium bicarbonate, sodium hydroxide, potassium hydroxide and combinations thereof.

14. The method of claim 1, wherein the method further comprises a step of replacing one or more of the W group and W' group with hydrogen after the heating step.

15. A monomer compound comprising the following formula: 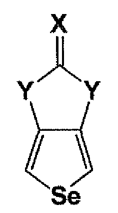 wherein Y comprises NH or O; and X comprises O or S.

16. The monomer of claim 14, wherein the monomer comprises at least one member selected from the group consisting of 1 H-selenolo[3,4-d]imidazol-2(3H)-one, selenolo[3,4-d]-1,3-dioxolan-2-one, 1 H-selenolo[3,4-d]imidazol-2(3H)-thione, and selenolo[3,4-d]-1,3-dioxolan-2-thione,

17. A monomer compound comprising the following formula: 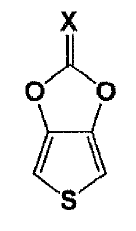 wherein X is S or O.

18. The monomer of claim 14, wherein the monomer comprises thieno[3,4-d]-1,3-dioxolan-2-one or thieno[3,4-d]-1,3-dioxolan-2-thione.

19. A composition comprising at least one monomer of Claim 18 and at least one catalyst.

20. A monomer compound derivative comprising the following formula: 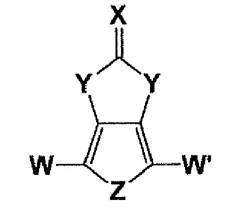 wherein Z comprises S or Se; Y comprises NH or O; X comprises O or S; W and W' comprises at least one member independently selected from the group consisting of halogen atoms, MgCl, MgBr, Mgl, ZnCl, ZnBr, Znl, Sn(R')₃, boronic acid, boronic ester, -CH=CHR", -OC₁₋₆ alkyl, -COOC₁₋₆alkyl, - S-COR"', -COR"', -C≡CH and polymerizable aromatic groups; R' is C₁₋₆ alkyl or -OC₁₋₆ alkyl; and R" and R'" are H or C₁₋₆alkyl.

21. The monomer compound derivative according to claim 20, wherein the polymerizable aromatic groups comprise at least one member selected from the group consisting of phenyl, naphthalene, pyrrole, dithiophene, thienothiophene, and thiophene.
